# EUROPEAN PATENT APPLICATION

(11) **EP 3 603 476 A1**
(43) Date of publication of application: **05.02.2020**
(21) Application number: 18771976.0
(22) Date of filing: 01.03.2018
(51) Int. Cl.: A61B 1/00, A61B 1/045, A61B 1/313, A61B 17/00

(54) **MEDICAL SYSTEM CONTROL DEVICE, MEDICAL SYSTEM CONTROL METHOD, AND MEDICAL SYSTEM**

(30) Priority: 24.03.2017 JP 2017059419
(71) Applicant: Sony Corporation, Tokyo 108-0075 (JP)
(72) Inventor: WADA, Seiji, Tokyo 108-0075 (JP); SAKAGUCHI, Tatsumi, Tokyo 108-0075 (JP); MAEDA, Takeshi, Tokyo 108-0075 (JP); MATSUURA, Kana, Tokyo 108-0075 (JP)
(74) Representative: D Young & Co LLP
(86) International application number: PCT/JP2018/007829
(87) International publication number: WO 2018/173681

(57) **Abstract**

[Problem]

To be able to visually recognize a treatment region from an angle different from a direction in which an endoscope is inserted when an inside of a human body is to be observed using the endoscope.

[Solving means]

According to the present disclosure, there is provided a control apparatus for a medical system, including a recognizing section that recognizes instruction information for indicating an area whose image is to be captured by an endoscope that is inserted into an inside of a human body through a trocar and captures an image of the inside of the human body, and a control information generator that generates first control information for turning the endoscope about the trocar and second control information for magnifying the area whose image is to be captured by the endoscope, based on the instruction information.

## Description

The present disclosure relates to a control apparatus for a medical system, a control method for a medical system, and a medical system.

### [Background Art]

In related art, PTL 1 referred to hereinbelow, for example, describes a technology for introducing a trocar into an abdominal wall, inserting a laparoscope into the hole in the trocar, and displaying an observed image of the abdominal cavity detected by the laparoscope on a TV monitor.

### [Citation List]

### [Patent Literature]

[PTL 1]
Japanese Patent Laid-open No. Hei 9-28713

### [Summary]

### [Technical Problems]

In a case where the laparoscope described in Patent Document 1 described above, particularly, a direct-vision scope, is used, the direct-vision scope captures a frontal image along the direction in which the direct-vision scope is inserted into the body of the patient. For visually recognizing a treatment region in the abdominal cavity from an angle different from the direction in which the direct-vision scope is inserted, it is general practice to bring a side surface or reverse side of the organ to the front and visually recognize same by pulling and rotating the organ with forceps.

According to such a method, however, it is assumed that the organ may be damaged when it is gripped, moved, or extended. According to the method, in addition, it is also assumed that the organ may be damaged when the gripped or moved organ contacts another organ.

Consequently, when the inside of a human body is to be observed using an endoscope, it is required to be able to visually recognize a treatment region from an angle different from the direction in which the endoscope is inserted.

### [Solution to Problems]

According to the present disclosure, there is provided a control apparatus for a medical system, including a recognizing section that recognizes instruction information for indicating an area whose image is to be captured by an endoscope that is inserted into an inside of a human body through a trocar and captures an image of the inside of the human body, and a control information generator that generates first control information for turning the endoscope about the trocar and second control information for magnifying the area whose image is to be captured by the endoscope, based on the instruction information.

According to the present disclosure, furthermore, there is provided a control method for a medical system, including recognizing instruction information for indicating an area whose image is to be captured by an endoscope that is inserted into an inside of a human body through a trocar and captures an image of the inside of the human body, and generating first control information for turning the endoscope about the trocar and second control information for magnifying the area whose image is to be captured by the endoscope, based on the instruction information.

According to the present disclosure, moreover, there is provided a medical system including a control apparatus for the medical system, including a recognizing section that recognizes instruction information for indicating an area whose image is to be captured by an endoscope that is inserted into an inside of a human body through a trocar and captures an image of the inside of the human body, and a control information generator that generates first control information for turning the endoscope about the trocar and second control information for magnifying the area whose image is to be captured by the endoscope, based on the instruction information, a support arm device that supports the endoscope, controlled based on the first control information, and the endoscope controlled based on the second control information.

### [Advantageous Effect of Invention]

According to the present disclosure, as described hereinbefore, it is possible to visually recognize a treatment region from an angle different from the direction in which an endoscope is inserted, when the inside of a human body is to be observed using the endoscope.

The above effect is not necessarily limitative, but there may be offered any of the effect indicated in the present description or other effect that can be grasped from the present description, together with or in addition to the above effect.

### [Brief Description of Drawings]

[FIG. 1]

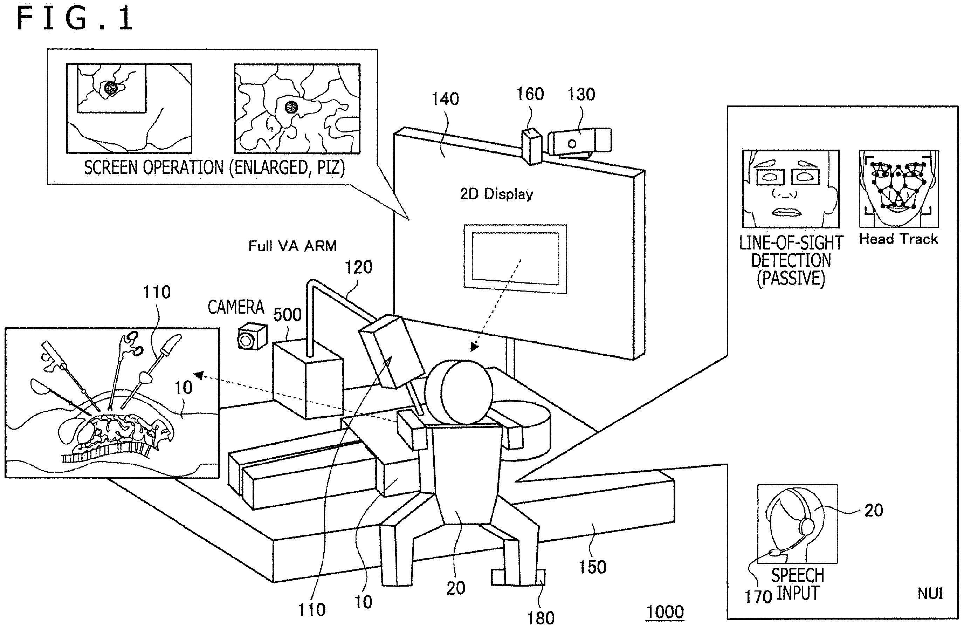

   FIG. 1 is a schematic diagram illustrating the general configuration of a surgery system according to an embodiment of the present disclosure.
[FIG. 2]

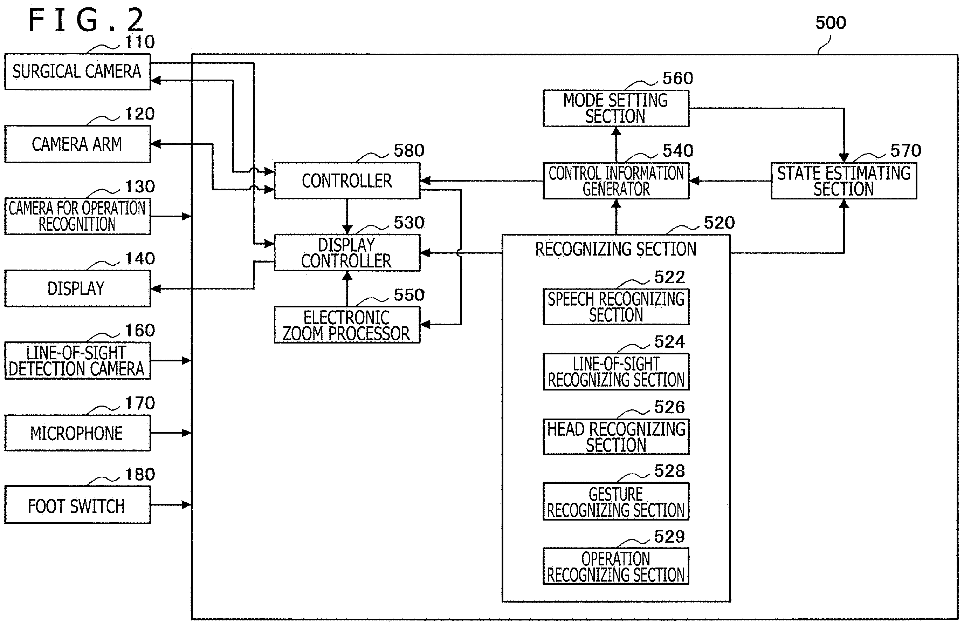

   FIG. 2 is a block diagram illustrating the configuration of a system including a control apparatus.
[FIG. 3A]

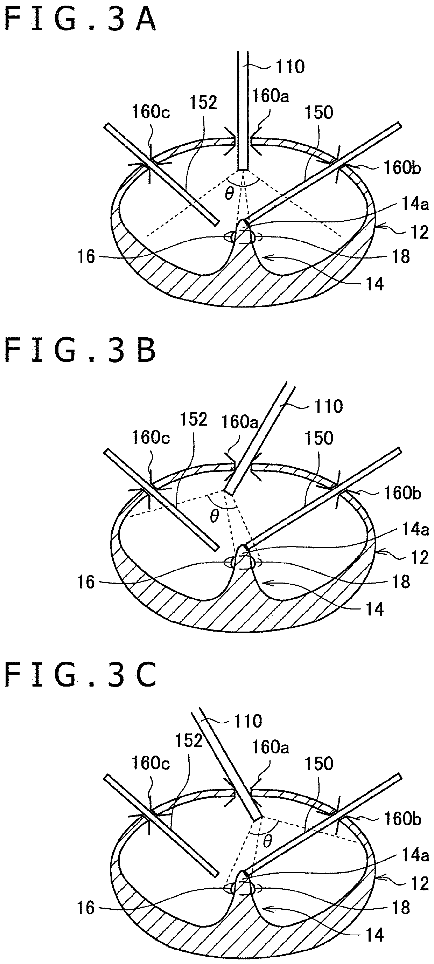

   FIG. 3A is a schematic cross-sectional view of an abdominal region of a patient, illustrating a state in which a surgical camera (direct-vision scope), and a forceps are inserted into the abdominal region.
[FIG. 3B]
   FIG. 3B is a schematic cross-sectional view of the abdominal region of the patient, illustrating a state in which the surgical camera (direct-vision scope), and the forceps are inserted into the abdominal region.
[FIG. 3C]
   FIG. 3C is a schematic cross-sectional view of the abdominal region of the patient, illustrating a state in which the surgical camera (direct-vision scope), and the forceps are inserted into the abdominal region.
[FIG. 4A]

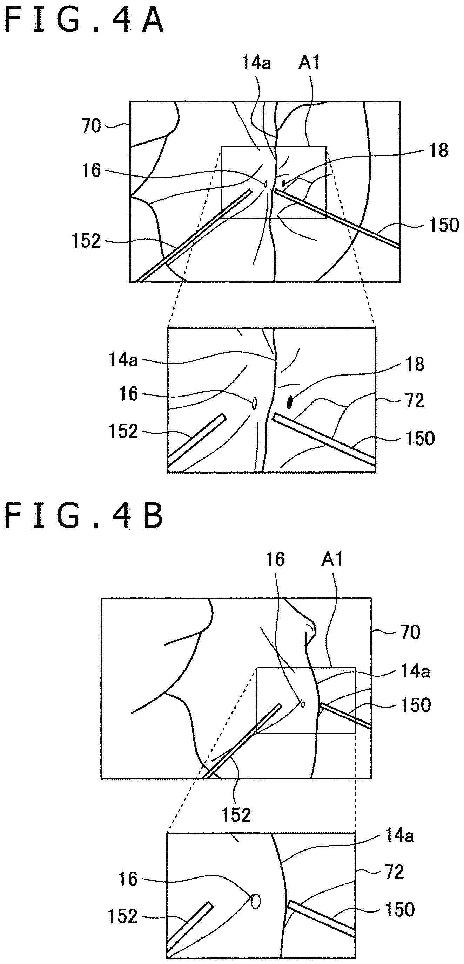

   FIG. 4A is a schematic view illustrating an image captured by the surgical camera in the state illustrated in FIG. 3A.
[FIG. 4B]
   FIG. 4B is a schematic view illustrating an image captured by the surgical camera in the state illustrated in FIG. 3B.
[FIG. 4C]

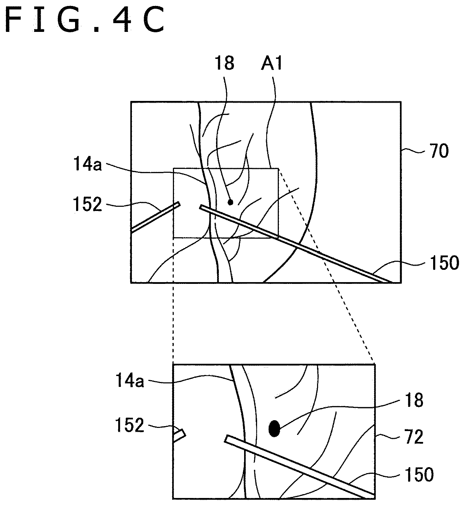

   FIG. 4C is a schematic view illustrating an image captured by the surgical camera in the state illustrated in FIG. 3C.
[FIG. 5]

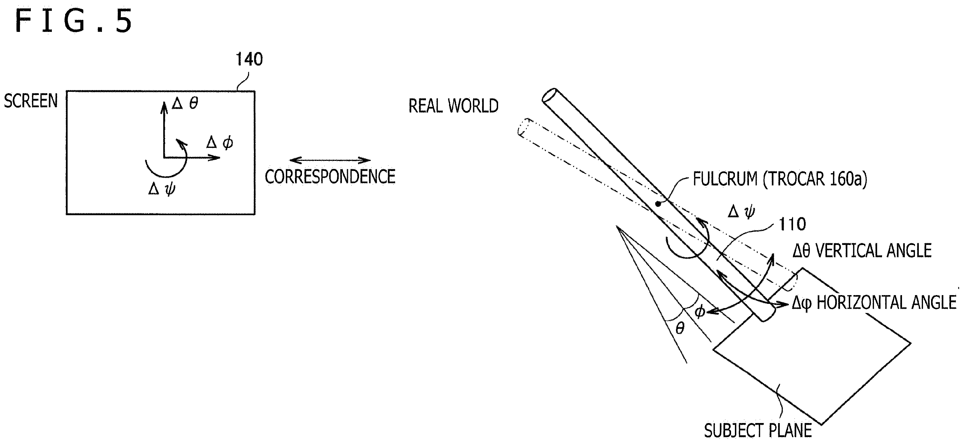

   FIG. 5 is a schematic view of an example of operation of a camera arm based on control information, illustrating a point-symmetry pivotal motion about a trocar.
[FIG. 6]

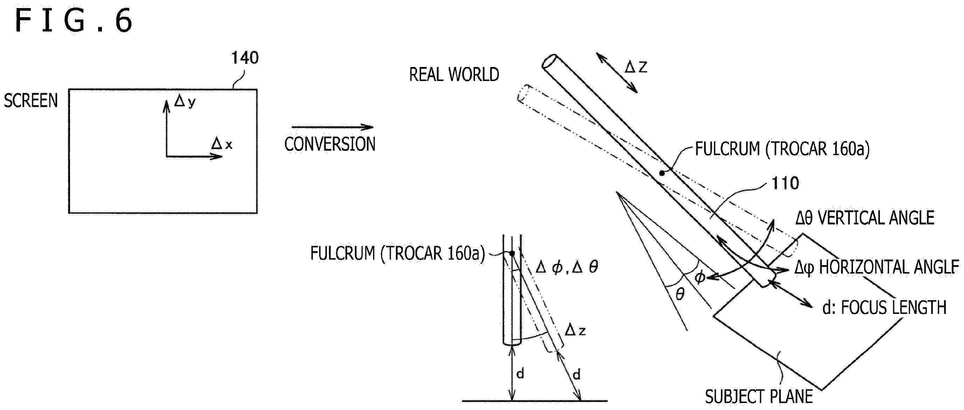

   FIG. 6 is a schematic view of an example of operation of the camera arm based on control information, illustrating an example of a zooming motion as well as the pivotal motion.

### [Description of Embodiment]

A preferred embodiment of the present disclosure will hereinafter be described in detail below with reference to the accompanying drawings. In the present description and drawings, those components having substantially identical functions and arrangements are denoted by identical reference signs, and their redundant description will be omitted.

The description will be given in the following sequence.
1. Example of Configuration of Surgical System
2. Method of Observing Side Surface of Treatment Region Using Direct-Vision Scope
3. Example of Operation of Camera Arm Based On Control Information

### 1. Example of Configuration of Surgical System

First, the general configuration of a surgical system 1000 according to an embodiment of the present disclosure will be described below with referred to FIG. 1. The surgical system 1000 according to the present embodiment is concerned with a system for performing a surgical operation with a laparoscope. According to laparoscopic surgery, a plurality of holes is made in the abdominal region of a patient 10, a surgical instrument such as forceps, an aspirator, an electrosurgical knife, or the like and a surgical camera 110 are inserted through the holes, and the abdominal region is operated on with the surgical tool while the inside of the abdominal region is being visually recognized with the surgical camera 110. FIG. 1 is a schematic view illustrating the positional relationship between the patient 10, a surgeon 20, and pieces of medical equipment in a system of laparoscopic surgery using the surgical camera 110. The surgeon 20 corresponds to an operation doctor, a scopist, or the like.

The surgical system 1000 includes the surgical camera 110, a camera arm 120 (support arm device), a movement recognition camera 130, a display 140, a surgical bed 150, a line-of-sight detection camera 160, a microphone 170, a foot switch 180, and a control apparatus 500. The surgical camera 110 is a device such as a 3D camera or the like held by the camera arm 120. The surgical camera 110 according to the present embodiment is constructed as a laparoscope, especially a direct-vision scope, and is inserted into the body of the patient 10 and captures an image of the inside of the body. The surgical camera 110 sends the captured image as a surgical-field image to the control apparatus 500.

The camera arm 120 holds the surgical camera 110 and controls the position and angle of the surgical camera 110. The movement recognition camera 130 is a 2D camera, for example, and is disposed on the display 140. The movement recognition camera 130 captures an image of the surgeon 20 and recognizes a movement of the surgeon 20. In order for the movement recognition camera 130 to recognize a movement of the surgeon 20 with ease, a marker may be mounted on the surgeon 20. The movement recognition camera 130 sends a captured 2D image as a surgeon image to the control apparatus 500.

The display 140 includes a relatively large screen and is disposed in a position relatively spaced from the surgeon 20. In the example illustrated in FIG. 1, the display 140 is disposed in a position that faces the surgeon 20 across the surgical bed 150. The display 140 displays a surgical-field image, etc. sent from the control apparatus 500.

The control apparatus 500 sets a manual mode or a hands-free mode as an operation mode. The manual mode is a mode for controlling the surgical system 1000 based on an input entered by the hand of the surgeon 20, e.g., a force applied to the camera arm 120, an operation of operation buttons, not illustrated, disposed in various portions. The hands-free mode is a mode for controlling the surgical system 1000 based on a contactless input such as speech, a motion or direction of a line of sight or a head, or a gesture, or a contact input such as contact of a foot with the foot switch 180, not entered by the hand of the surgeon 20.

Furthermore, the control apparatus 500 receives a surgeon image sent from the movement recognition camera 130, detects the position of the head of the surgeon 20 in the surgeon image, and detects the movement of the head (head tracking), and the direction of the head. Moreover, the control apparatus 500 recognizes a gesture of the surgeon 20 from the surgeon image.

Furthermore, the control apparatus 500 receives information representing the direction of the line of sight of the surgeon 20 sent from the line-of-sight detection camera 160, and recognizes the position of the line of sight on the screen of the display 140 based on the information and the position and direction of the head of the surgeon 20. In addition, the control apparatus 500 receives speech sent from the microphone 170 and performs speech recognition on the speech. Moreover, the control apparatus 500 receives an operation signal representing an operation on the foot switch 180 sent from the foot switch 180, and recognizes the content of the operation on the foot switch 180 based on the operation signal.

Furthermore, in a case where the operation mode is the hands-free mode, the control apparatus 500 uses, as input information, the movement and direction of the head of the surgeon 20, the gesture of the surgeon 20, the line-of-sight information representing the position of the line of sight on the screen of the display 140, the result of the speech recognition, the sound volume, and the content of the operation on the foot switch 180. The control apparatus 500 recognizes an instruction from the surgeon 20 and a state of the surgeon 20 based on the input information.

Depending on the state of the surgeon 20, the control apparatus 500 permits the instruction from the surgeon 20. Dependent on the permitted instruction, the control apparatus 500 controls image capturing of the surgical camera 110, actuation of the camera arm 120, display on the display 140, and changes the operation mode.

The microphone 170 is mounted on the surgeon 20. The microphone 180 acquires speech in the periphery including speech, etc. of the surgeon 20, and sends the acquired speech to the control apparatus 500. The foot switch 180 is disposed in the periphery of the surgeon 20, and is operated by contact of the leg of the surgeon 20. The foot switch 180 sends an operation signal representing an operation of the leg of the surgeon 20 to the control apparatus 500.

In the surgical system 1000 constructed above, the surgeon 20 lays the patient 10 on the surgical bed 150, and performs a surgical operation while seeing the surgical-field image displayed on the display 140.

Furthermore, for changing the operation mode, image-capturing conditions of the surgical camera 110, the position and angle of the surgical camera 110, the display on the display 140, etc., the surgeon 20 enters a contactless input or an input by way of contact of the foot. Therefore, the surgeon 20 can enter an input while gripping the surgical instrument.

In addition, any desired methods can be employed as a method of detecting the line of sight, a method of detecting the movement and direction of the head of the surgeon 20 and the gesture thereof, a method of acquiring the speech, and so on.

In the system 1000 illustrated in FIG. 1, as described hereinbefore, the camera arm 120 grips the surgical camera 110, and the surgeon 20 controls the posture of the surgical camera 110 while seeing the image captured by the surgical camera 110 on the display 140.

According to the present embodiment, control information and additional information are added to the image from the surgical camera 110 and displayed on the display 140, so that the surgeon 20 can visually recognize the image. FIG. 2 is a block diagram illustrating the configuration of the system 1000 including the control apparatus 500. As illustrated in FIG. 2, the surgical camera 110, the camera arm 120, the movement recognition camera 130, the display 140, the line-of-sight detection camera 160, the microphone 170, and the foot switch 180 are connected to the control apparatus 500. In FIG. 2, the configuration of the surgical camera 110 includes a camera control unit (CCU) for controlling the focal position, focal length, etc. of the surgical camera 110.

The control apparatus 500 adjusts the spatial position and angle of the surgical camera 110 in response to an instruction from the surgeon 20. According to the present embodiment, in a case where the surgeon 20 is to issue an instruction, the surgeon 20 may issue a hands-free instruction based on the direction of the line of sight, the movement of the head, the speech, or the like, in addition to an instruction via the foot switch 180. Instruction information according to the instruction from the surgeon 20 is sent to the control apparatus 500.

The control apparatus 500 includes a recognizing section 520, a display controller 530, a control information generator 540, an electronic zoom processor 550, a mode setting section 560, a state estimating section 570, and a controller 580. The recognizing section 520 includes a speech recognizing section 522, a line-of-sight recognizing section 524, a head recognizing section 526, a gesture recognizing section 528, and an operation recognizing section 529. The components of control apparatus 500 illustrated in FIG. 2 may be implemented by circuits (hardware), or a central arithmetic/processing unit such as a CPU or the like and programs (software), that enable the central arithmetic/processing unit to function.

The speech recognizing section 522 performs speech recognition on the speech sent from the microphone 170, and recognizes an utterance as a contactless input from the surgeon 20. Moreover, the speech recognizing section 522 recognizes a volume of speech sent from the microphone 170 as a contactless input from the surgeon 20. The speech recognizing section 522 supplies the utterance and the volume of speech as the result of the speech recognition as input information to the control information generator 540.

The line-of-sight recognizing section 524 recognizes the position of a line of sight on the screen of the display 140 as a contactless input from the surgeon 20, based on the information representing the direction of the line of sight sent from the line-of-sight detection camera 160 and the position and direction of the head recognized by the head recognizing section 526. The line-of-sight recognizing section 524 supplies line-of-sight-position information representing the position of the line of sight as input information to the control information generator 540, the state estimating section 570, and the display controller 530.

The head recognizing section 526 recognizes the position, movement, and direction of the head of the surgeon 20 as a contactless input from the surgeon 20 by detecting the position of the surgeon 20 from the surgeon image sent from the movement recognition camera 130. The head recognizing section 526 supplies the movement and direction of the head as input information to the control information generator 540 and the state estimating section 570. In addition, the head recognizing section 526 supplies the position and direction of the head to the line-of-sight recognizing section 524.

The gesture recognizing section 528 recognizes an input of the gesture of the surgeon 20 as a contactless input from the surgeon 20 from the surgeon image sent from the movement recognition camera 130. The gesture recognizing section 528 supplies the gesture of the surgeon 20 as input information to the control information generator 540.

The operation recognizing section 529 receives an operation signal sent from the foot switch 180, and recognizes the content of the operation on the foot switch 180 as a contact input from the surgeon 20. The operation recognizing section 529 supplies operation information representing the content of the operation as input information to the control information generator 540.

The control information generator 540 recognizes an instruction from the surgeon 20 based on the input information supplied from the recognizing section 520, and generates control information for controlling the camera arm 120 based on the instruction. If the recognized instruction is an instruction for changing the operation mode, then the control information generator 540 notifies the mode setting section 560 that sets a mode based on the instruction.

The mode setting section 560 sets the operation mode to the manual mode or the hands-free mode according to the instruction supplied from the control information generator 540. The mode setting section 560 supplies the set operation mode to the state estimating section 570.

In a case where the operation mode supplied from the mode setting section 560, then the state estimating section 570 estimates a state of the surgeon 20 based on the input information supplied from the recognizing section 520. The state estimating section 570 notifies the control information generator 540 of the estimated state.

The controller 580 executes an instruction based on control information supplied from the control information generator 540. Specifically, if control information supplied from the control information generator 540 is control information about controlling image capturing of the surgical camera 110, then the controller 580 controls image capturing of the surgical camera 110 according to the control information. Various image capturing functions such as an electronic zooming function of the surgical camera 110 are thereby controlled.

Furthermore, in a case where control information supplied from the control information generator 540 is an instruction about controlling actuation of the camera arm 120, then the controller 580 controls actuation of the camera arm 120 according to the control information. According to an example, the camera arm 120 includes a plurality of joints and actuators incorporated in the respective joints. The actuators in the respective joints are actuated under the control of the controller 580 to realize a movement of the camera arm 120 according to the control information. In a case where an instruction supplied from the control information generator 540 is control information about controlling display on the display 140, then the controller 580 supplies the control information to the display controller 530 thereby to control the display controller 530.

The display controller 530 performs a processing sequence for displaying a surgical-field image sent from the surgical camera 110 on the display 140. Moreover, in a case where control information supplied from the controller 580 is an annotation display instruction, then the display controller 530 superposes a mark over a position corresponding to the line of sight of the surgeon 20 in the surgical-field image sent from the surgical camera 110, based on line-of-sight-position information supplied from the line-of-sight recognizing section 524. Then, the display controller 530 supplies the surgical-field image with the mark superposed thereon to the display 140, enabling it to display the surgical-field image.

Furthermore, in a case where an instruction supplied from the controller 580 is a menu display instruction for displaying a GUI, (Graphical User Interface), such as menu buttons, on the display 140, then the display controller 530 superposes an image of the GUI on the surgical-field image sent from the surgical camera 110. The display controller 530 supplies the surgical-field image with the GUI superposed thereon to the display 140, enabling it to display the surgical-field image.

For example, in a case where the result of speech recognition in input information is "zoom-in" and the line-of-sight-position information represents a position on the screen of the display 140, then the control information generator 540 recognizes that the instruction from the surgeon 20 is an instruction for controlling the surgical camera 110 to capture a zoom-in image including at its center a subject corresponding to the position of a line of sight represented by the line-of-sight-position information. Then, the control information generator 540 generates control information for executing the recognized instruction.

Similarly, if the result of speech recognition in input information is "zoom-out" and the line-of-sight-position information represents a position on the screen of the display 140, then the control information generator 540 recognizes that the instruction from the surgeon 20 is an instruction for controlling the surgical camera 110 to capture a zoom-out image including at its center a subject corresponding to the position of a line of sight represented by the line-of-sight-position information. Then, the control information generator 540 generates control information for executing the recognized instruction.

If the result of speech recognition in input information is "focus" and the line-of-sight-position information represents a position on the screen of the display 140, then the control information generator 540 recognizes that the instruction from the surgeon 20 is an instruction for performing focus control on the surgical camera 110 to focus itself on a subject corresponding to the position of a line of sight represented by the line-of-sight-position information. Then, the control information generator 540 generates control information for executing the recognized instruction.

As described above, it is possible for the surgeon 20 to enter the content of image capturing control with speech suitable for instruction inputs and to enter a position required for image capturing control with a line of sight suitable for position inputs. Therefore, the surgeon 20 can easily enter instructions with respect to image capturing control.

Moreover, if the result of speech recognition in input information is "pivot," the line-of-sight-position information represents a position on the screen of the display 140, the line-of-sight-position information does not change chronologically, the movement of the head of the surgeon 20 represents a motion, and the operation information represents a depression of the foot switch 180, then the control information generator 540 recognizes that the instruction from the surgeon 20 is an instruction for controlling the camera arm 120 to cause the surgical camera 110 to make a pivotal motion according to the movement of the head. Then, the control information generator 540 generates control information for executing the recognized instruction.

If the result of speech recognition in input information is "slide," the movement of the head of the surgeon 20 represents a rotation, the line-of-sight-position information represents a position on the screen of the display 140, the position represented by the line-of-sight-position information changes chronologically in a direction that is the same as the direction of rotation of the head, and the operation information represents a depression of the foot switch 180, then the control information generator 540 recognizes that the instruction from the surgeon 20 is an instruction for controlling the camera arm 120 to cause the surgical camera 110 to make a sliding motion according to the position of the line of sight. Then, the control information generator 540 generates control information for executing the recognized instruction.

In addition, since a pivotal motion instruction and a sliding motion instruction are instructions about controlling actuation of the camera arm 120, the kind of these instructions is classified under camera arm control.

### 2. Method of Observing Side Surface of Treatment Region Using Direct-Vision Scope

Incidentally, if a laparoscope, especially a direct-vision scope, is used as the surgical camera 110, then the direct-vision camera captures a frontal image along the direction in which the direct-vision scope is inserted into the body of the patient 10. Here, when a three-dimensional object such as an organ is to be visually recognized, there may arise a situation where the three-dimensional object should be visually recognized in the direction of a side surface thereof. According to a general method, the object to be visually recognized is held in a frontal region, the camera is fixed in position, and the organ is moved using forceps, so that a side surface of the three-dimensional object such as the organ can be visually recognized. According to this method, however, it is assumed that the organ may be damaged when it is gripped, moved, or extended. According to the method, in addition, it is also assumed that the organ may be damaged when the gripped or moved organ contacts another organ.

According to the present embodiment, the surgical camera 110 is placed such that an object such as an organ or the like is positioned at an end of the viewing angle of the surgical camera 110, the tip end of the lens barrel of the surgical camera 110 is swung to cut off an image of the range of the object, and the image is magnified by electronic zooming, so that the angle at which the object is visually recognized is changed without changing the range in which the object is visually recognized. In this manner, the side surface of the organ can be visually recognized though the direct-vision scope is used.

FIGS. 3A through 3C and FIGS. 4A through 4C are schematic views illustrating a case in which a side surface of an organ is visually recognized using a direct-vision scope by a process according to the present embodiment. FIGS. 3A through 3C illustrate a cross section of an abdominal region 12 of the patent 10, in which the surgical camera (direct-vision scope), 110 and forceps 150 and 152 are inserted in the abdominal region 12. According to endoscopic surgery, an abdominal wall is punctured by a plurality of tubular open pore instruments called trocars 160a, 160b and 160c, rather than performing laparotomy. Then, other surgical instruments such as the lens barrel of the surgical camera 110, the forceps 150 and 152, etc. are inserted through the trocars 160a, 160b and 160c into the body cavity in the patient 10.

FIGS. 3A through 3C each illustrate a state in which an internal organ 14 in the abdominal region 12 is gripped by the forceps 150 and lifted. As illustrated in FIGS. 3A through 3C, as the internal organ 14 is lifted, the internal organ 14 is deformed, forming a protuberance 14a. In FIGS. 3A through 3C, the protuberance 14a is supposed to have areas 16 and 18 to be visually recognized by the surgeon 20 on side surfaces thereof.

FIG. 3A illustrates a state in which the protuberance 14a is positioned in a frontal region along the direction in which the surgical camera 110 is inserted into the abdominal region 12. FIG. 4A illustrates an image captured by the surgical camera 110 in the state illustrated in FIG. 3A. In FIG. 4A, an image 70 represents an original image captured by the surgical camera 110. In FIG. 4A, moreover, an image 72 represents an magnified image obtained by magnifying an area A1 in the image 70.

As illustrated in FIG. 4A, in a case where the protuberance 14a is positioned in the frontal region along the direction in which the surgical camera 110 is inserted, the surgical camera 110 is unable to capture frontal images of the areas 16 and 18 to be visually recognized by the operation doctor 10. Therefore, the areas 16 and 18 cannot be observed in detail by using the image 72 produced by magnifying the area A1 in the image 70.

FIG. 3B illustrates a state in which the surgical camera 110 has been angularly moved about the trocar 160a from the state in FIG. 3A by a pivotal motion of the surgical camera 110. When the surgical camera 110 is angularly moved, the protuberance 14a is positioned at an end of the viewing angle θ of the surgical camera 110. FIG. 4B illustrates an image captured by the surgical camera 110 in the state illustrated in FIG. 3B. In FIG. 4B, as with FIG. 4A, an image 70 also represents an original image captured by the surgical camera 110. In FIG. 4B, moreover, an image 72 represents a magnified image obtained by magnifying an area A1 in the image 70.

As illustrated in FIG. 4B, because the protuberance 14a is positioned at the end of the viewing angle θ of the surgical camera 110, a more detailed image of a side surface of the protuberance 14a than in FIG. 4A is captured. Since a frontal image of the area 16 on the left side of the ridgeline of the protuberance 14a is captured, as indicated by the image 72 in FIG. 4B, it is possible to reliably observe the area 16 that could not be obtained in detail in FIG. 4A.

FIG. 3C illustrates a state in which the surgical camera 110 has been angularly moved about the trocar 160a from the state in FIG. 3B in a direction opposite FIG. 3B by a pivotal motion of the surgical camera 110. In FIG. 3C, too, when the surgical camera 110 is angularly moved, the protuberance 14a is positioned at an end of the viewing angle θ of the surgical camera 110. FIG. 4C illustrates an image captured by the surgical camera 110 in the state illustrated in FIG. 3C. In FIG. 4C, as with FIGS. 4A and 4B, an image 70 also represents an original image captured by the surgical camera 110. In FIG. 4C, moreover, an image 72 represents a magnified image obtained by magnifying an area A1 in the image 70.

As illustrated in FIG. 4C, because the protuberance 14a is positioned at the end of the viewing angle θ of the surgical camera 110, a more detailed image of a side surface of the protuberance 14a than in FIG. 4A is captured. Since a frontal image of the area 18 on the right side of the ridgeline of the protuberance 14a is captured, as indicated by the image 72 in FIG. 4C, it is possible to reliably observe the area 18 that could not be obtained in detail in FIG. 4A.

### 3. Example of Operation of Camera Arm Based On Control Information

Next, operation of the camera arm 120 based on control information will be described below. Typical commands for the camera arm 120 include a move command for translating the surgical camera 110, a pivot command for turning the surgical camera 110 with respect to an object, and a pan/tilt/zoom command for the surgical camera 110. Panning/tilting/rolling motions, a zooming motion, and a moving motion of the surgical camera 110 are combined to realize a turning camera motion as seen from the user. FIG. 5 is a schematic view of an example of operation of the camera arm 120 based on control information, illustrating a point-symmetry pivotal motion about the trocar 160a illustrated in FIGS. 3A through 3C. An example in which the surgical camera 110 is turned from the state illustrated in FIG. 3A to the state illustrated in FIG. 3B will hereinafter be described. As described hereinbefore, the surgeon 20 issues an instruction by operating the foot switch 180 or issues a hands-free instruction by directing its line of sight or moving its head while seeing the screen of the display 140, thereby generating control information for controlling the camera arm 120. Consequently, control information for controlling actual movement of the camera arm 120 is generated based on instruction information based on the amount of operation on the screen illustrated in FIG. 6.

As illustrated in FIG. 5, the surgeon 20 enters a screen horizontal angle Δφ, a screen vertical angle Δθ, and a screen angle Δϕ as instruction information while referring to the screen of the display 140. Here, the screen horizontal angle Δφ is an angle change in a horizontal direction, the screen vertical angle Δθ is an angle change in a vertical direction, and the screen angle Δϕ is a rotational angle about the longitudinal axis of the surgical camera 110.

A hands-free operation of the surgeon 20 is basically the same as with the pivotal motion described hereinbefore. The surgeon 20 utters a pivotal motion about the trocar 160a and moves the position of its line of sight on the display 140 by a distance corresponding to the screen horizontal angle Δφ.

If an input (instruction information), on the screen is PTR (Δφ, Δθ, Δϕ), then the input is converted into an actual motion of the camera arm 120, and control information for controlling the camera arm 120 becomes PTR (-Δφ, -Δθ, -Δϕ). The conversion may be carried out using a preset table. Here, the signs of Δφ and Δθ are reversed because the motion on the screen and the motion of the tip end of the surgical camera 110 are opposite as the surgical camera 110 is turned about the trocar 160a for the transition from FIG. 3A to FIG. 3B. As described above, for moving the surgical camera 110 from the state of FIG. 3A to the state of FIG. 3B, for example, the screen horizontal angle Δφ is mainly changed thereby to turn the surgical camera 110 about the trocar 160a.

In this manner, the surgical camera 110 is turned from the state illustrated in FIG. 3A to the state illustrated in FIG. 3B. The screen of the display 140 transitions from the image 70 illustrated in FIG. 4A to the image 70 illustrated in FIG. 4B. In the image 70 illustrated in FIG. 4B, since the protuberance 14a is positioned in the end of the viewing angle θ of the surgical camera 110, in a case where the surgeon 20 observes the area 16 on the left side of the ridgeline of the protuberance 14a, the surgeon 20 is unable to visually recognize the area 16 at the center of the screen. In the image 70 illustrated in FIG. 4B, furthermore, as the turning motion of the surgical camera 110 has increased the distance between the tip end of the surgical camera 110 and the protuberance 14a, the area 16 is scaled down.

Therefore, the control information generator 540 generates control information for magnifying the area A1 of the image 70 illustrated in FIG. 4B as well as the control information for turning the surgical camera 110. As a result, the camera arm 120 controlled based on the control information turns the surgical camera 110 from the state illustrated in FIG. 3A to the state illustrated in FIG. 3B. In addition, the surgical camera 110 is controlled based on the control information to perform a processing sequence to magnify the area A1 of the image 70 illustrated in FIG. 4B to generate the image 72 according to the electronic zooming function of the surgical camera 110. Consequently, the image 72 where the area 16 to be observed is displayed at the center of the scree of the display 140 is generated. The larger the amount of a turning motion of the surgical camera 110 is, the larger the distance between the tip end of the surgical camera 110 and the protuberance 14a becomes, so that the area 16 is more scaled down. Therefore, it is desirable to generate control information such that the larger the amount of a turning motion of the surgical camera 110 is, the higher the magnification by the electronic zooming function is. The relationship between the amounts of turning motions of the surgical camera 110 and the magnifications by the electronic zooming function may be prescribed by a preset table or the like.

The processing sequence to magnify the area A1 of the image 70 to generate the image 72 may be carried out by the control apparatus 500. In this case, the control apparatus 500 acquires the image information of the image 70 from the surgical camera 110. Then, the electronic zoom processor 550 performs an electronic zooming process on the image 70 to magnify the area A1 of the image 70 to generate the image 72. The image 72 is displayed on the display 140 by the display controller 530.

FIG. 6 is a schematic view of an example of operation of the camera arm 120 based on control information, illustrating an example in which a zooming motion is made to bring the surgical camera 110 closely to the subject to make a moving motion to move the subject in a horizontal direction, as well as the pivotal action illustrated in FIG. 5.

As illustrated in FIG. 6, the surgeon 20 enters a horizontal movement distance Δx and a vertical movement distance Δy on the screen as instruction information while seeing the screen of the display 140. Δx is indicated as a ratio (%) to the horizontal length of the screen as a reference, and Δy is indicated as a ratio (%) to the vertical length of the screen as a reference.

The control information generator 540 converts the horizontal movement distance Δx and the vertical movement distance Δy into a horizontal angle Δφ, a vertical angle Δθ, and an axial movement distance Δz for the surgical camera 110. The conversion may be carried out using a preset table. Based on the conversion, if the input (instruction information), on the screen is MOVE (Δx, Δy), then the control information for controlling the camera arm 120 becomes PTR (-Δφ, -Δθ), MOVE (Δz). Because of the above control information, although the distance between the tip end of the surgical camera 110 and the subject increases as the surgical camera 110 is turned, the surgical camera 110 is axially moved. The surgical camera 110 is hereby brought closely to the subject by a distance commensurate with the increase in the distance, so that the value of the distance d between the tip end of the surgical camera 110 and the subject remains unchanged. As a consequence, although the surgical camera 110 makes a turning motion, since the size of the subject on the display 140 remains unchanged, the surgeon 20 who visually recognizes the display 140 is given a visual effect as if the surgical camera 110 was moved in a horizontal direction.

According to the present embodiment, as described hereinbefore, for observing the inside of a human body using the surgical camera (direct-vision scope), 110, the surgical camera 110 is turned so that the subject is positioned at the end of the viewing angle of the surgical camera 110 and the area of the subject that has moved to a peripheral region of the screen is displayed at a magnified ratio. When the inside of a human body is to be observed using a direct-vision scope, it is thus possible to visually recognize a treatment region from an angle different from the direction in which the direct-vision scope is inserted.

While the preferred embodiment of the present disclosure has heretofore been described in detail above with reference to the accompanying drawings, the technical scope of the present disclosure is not limited to the above example. Those who have ordinary knowledge in the technical field of the present disclosure are obviously able to envisage various changes or revisions within the scope of the technical idea stated in the scope of the claims for patent, and it should be understood that such various changes or revisions will inevitably fall within the technical scope of the present disclosure.

The advantages stated in the present description are only explanatory or illustrative, but not limitative. In other words, the technology according to the present disclosure can offer other advantages that are obvious to those skilled in the art from the above description, together with or in addition to the above advantages.

It is to be noted that, the following arrangements belong to the technical scope of the present disclosure.
(1) A control apparatus for a medical system, including:
   a recognizing section that recognizes instruction information for indicating an area whose image is to be captured by an endoscope that is inserted into an inside of a human body through a trocar and captures an image of the inside of the human body; and
   a control information generator that generates first control information for turning the endoscope about the trocar and second control information for magnifying the area whose image is to be captured by the endoscope, based on the instruction information.
(2) The control apparatus for the medical system according to (1), in which
   the area whose image is to be capture is moved toward an end of a viewing angle of the endoscope by the turning of the endoscope based on the first control information.
(3) The control apparatus for the medical system according to (2), in which
   an image of an area in a depthwise direction of a subject is captured by the endoscope before the area whose image is to be captured is moved, when the area whose image is to be captured is moved toward the end of the viewing angle of the endoscope.
(4) The control apparatus for the medical system according to any one of (1) to (3), in which
   the larger the turning of the endoscope based on the first control information is, the higher the magnification of the area whose image is to be captured based on the second control information becomes.
(5) The control apparatus for the medical system according to any one of (1) to (4), in which
   a support arm device that supports the endoscope is controlled based on the first control information.
(6) The control apparatus for the medical system according to any one of (1) to (5), in which
   the endoscope is controlled to magnify the area whose image is to be captured based on the second control information.
(7) The control apparatus for the medical system according to any one of (1) to (5), further including:
   an electronic zoom processor that performs an electronic zooming process for magnifying the area whose image is to be captured based on the second control information.
(8) The control apparatus for the medical system according to any one of (1) to (7), in which
   the recognizing section recognizes the instruction information sent from a foot switch operated by a user, a movement recognition camera for detecting movement of the head of the user, a line-of-sight detection camera for detecting the direction of the line of sight of the user, or a microphone for acquiring speech information from an utterance of the user.
(9) A control method for a medical system, including:
   recognizing instruction information for indicating an area whose image is to be captured by an endoscope that is inserted into an inside of a human body through a trocar and captures an image of the inside of the human body; and
   generating first control information for turning the endoscope about the trocar and second control information for magnifying the area whose image is to be captured by the endoscope, based on the instruction information.
(10) A medical system including:
   a control apparatus for the medical system, including a recognizing section that recognizes instruction information for indicating an area whose image is to be captured by an endoscope that is inserted into an inside of a human body through a trocar and captures an image of the inside of the human body, and a control information generator that generates first control information for turning the endoscope about the trocar and second control information for magnifying the area whose image is to be captured by the endoscope, based on the instruction information;
   a support arm device that supports the endoscope, controlled based on the first control information; and
   the endoscope controlled based on the second control information.

### [Reference Signs List]

- 110: Surgical camera
- 120: Camera arm
- 130: Movement recognition camera
- 160: Line-of-sight detection camera
- 170: Microphone
- 180: Foot switch
- 500: Control apparatus
- 520: Recognizing section
- 540: Control information generator

## Claims

1. A control apparatus for a medical system, comprising:
a recognizing section that recognizes instruction information for indicating an area whose image is to be captured by an endoscope that is inserted into an inside of a human body through a trocar and captures an image of the inside of the human body; and
a control information generator that generates first control information for turning the endoscope about the trocar and second control information for magnifying the area whose image is to be captured by the endoscope, based on the instruction information.

2. The control apparatus for the medical system according to claim 1, wherein
the area whose image is to be captured is moved toward an end of a viewing angle of the endoscope by the turning of the endoscope based on the first control information.

3. The control apparatus for the medical system according to claim 2, wherein
an image of an area in a depthwise direction of a subject is captured by the endoscope before the area whose image is to be captured is moved, when the area whose image is to be captured is moved toward the end of the viewing angle of the endoscope.

4. The control apparatus for the medical system according to claim 1, wherein
the larger the turning of the endoscope based on the first control information is, the higher the magnification of the area whose image is to be captured based on the second control information becomes.

5. The control apparatus for the medical system according to claim 1, wherein
a support arm device that supports the endoscope is controlled based on the first control information.

6. The control apparatus for the medical system according to claim 1, wherein
the endoscope is controlled to magnify the area whose image is to be captured based on the second control information.

7. The control apparatus for the medical system according to claim 1, further comprising:
an electronic zoom processor that performs an electronic zooming process for magnifying the area whose image is to be captured based on the second control information.

8. The control apparatus for the medical system according to claim 1, wherein
the recognizing section recognizes the instruction information sent from a foot switch operated by a user, a movement recognition camera for detecting movement of the head of the user, a line-of-sight detection camera for detecting the direction of the line of sight of the user, or a microphone for acquiring speech information from an utterance of the user.

9. A control method for a medical system, comprising:
recognizing instruction information for indicating an area whose image is to be captured by an endoscope that is inserted into an inside of a human body through a trocar and captures an image of the inside of the human body; and
generating first control information for turning the endoscope about the trocar and second control information for magnifying the area whose image is to be captured by the endoscope, based on the instruction information.

10. A medical system comprising:
a control apparatus for the medical system, including a recognizing section that recognizes instruction information for indicating an area whose image is to be captured by an endoscope that is inserted into an inside of a human body through a trocar and captures an image of the inside of the human body, and a control information generator that generates first control information for turning the endoscope about the trocar and second control information for magnifying the area whose image is to be captured by the endoscope, based on the instruction information;
a support arm device that supports the endoscope, controlled based on the first control information; and
the endoscope controlled based on the second control information.
